# EUROPEAN PATENT APPLICATION

(11) **EP 0 943 336 A1**
(43) Date of publication of application: **22.09.1999**
(21) Application number: 97939149.7
(22) Date of filing: 03.09.1997
(51) Int. Cl.: A61K 38/23, A61K 38/28, A61K 38/24, A61K 38/25, A61K 38/27, A61K 38/26, A61K 38/22, A61K 47/02, A61K 9/14, A61K 9/16

(54) **PEPTIDE-CONTAINING PHARMACEUTICAL COMPOSITIONS FOR ORAL ADMINISTRATION**

(30) Priority: 04.09.1996 JP 25220296; 04.03.1997 JP 6397297
(71) Applicant: DOTT RESEARCH LABORATORY, Yokohama-shi, Kanagawa 224 (JP)
(72) Inventor: YANAGAWA, Akira, Kanagawa 224-0051 (JP)
(74) Representative: Weisert, Annekäte, Dipl.-Ing. Dr.-Ing.
(86) International application number: JP9703081
(87) International publication number: WO9809645

(57) **Abstract**

The present invention provides a pharmaceutical composition for oral administration containing physiologically active peptide such as peptide hormones, opioid peptides, physiologically active proteins, enzyme proteins and the like, with an excellent stability and enhanced absorption via the oral route. More specifically, the invention provides a peptide-containing pharmaceutical composition for oral administration prepared as an enteric coated preparation, wherein an effective dose of physiologically active peptide is dispersed in a physiologically acceptable porous powdery or crystalline polyvalent metal compound carrier with a mean particle diameter of 500 µm or less, i.e., divalent or higher valency metal compound such as aluminum, calcium, magnesium, silicon, iron and zinc compounds.

The pharmaceutical composition for oral administration according to the present invention enhances the absorption of physiologically active peptides that normally show poor absorption via the oral route.

## Description

### Technical Field

The present invention relates to a peptide-containing pharmaceutical composition and more specifically, a pharmaceutical composition for oral administration that provides an excellent pharmaceutical stability of physiologically active peptides such as peptide hormones, physiologically active proteins and enzyme proteins, and enhances the absorption of the peptide contained in the composition after oral administration.

### Background Art

Various physiologically active peptides, including calcitonin, insulin, parathyroid hormone (PTH), human growth hormone (HGH), luteinizing hormone-releasing hormone (LH-RH), gonadotropin-releasing hormone (GnRH), and the like and their derivatives, are macromolecular compounds used for various medical purposes in clinical practice owing to their unique physiological actions.

These physiologically active peptides are subject to degradation by proteases in the digestive tract, and therefore show little absorption in the intact form through the intestinal mucosa. Hence, they are hard to administer orally and are limited to parenteral administration. However, administration via injection is undesirable because it causes pain to the patient, and this is particularly a problem when drugs need to be given repeatedly at regular intervals.

Accordingly, there has been demand for development of non-injectable preparations which can be administered safely and repeatedly, and which would allow self medication.

As one of such preparations, the present inventor has been investigating a powder preparation for nasal administration. Previously, the inventor found that a nasally administrable composition wherein physiologically active peptide is dispersed homogeneously in and adsorbed onto a specific carrier which he discovered shows enhanced absorbability of the peptide from the nasal mucosa, and he filed patent applications for the inventions based on the findings (e.g., US Patent Nos. 5574006 and 5603943).

The nasally administrable preparation proposed by the present inventor as above enhances the bioavailability of physiologically active peptides that are hard to administer via the oral route, and increases it to a similar level to that obtained with conventional injectable preparations, so the preparation has excellent clinical applicability. However, to improve the QOL of patients, development of orally administrable preparations is also desirable.

The present inventor extensively performed investigations towards this end, and eventually found that to enhance oral absorption of physiologically active peptides, physiologically acceptable porous powdery or crystalline polyvalent metal compounds having a mean particle diameter of 500 µm or less are useful as a carrier for drug delivery of those peptides.

That is, when physiologically active peptides such as calcitonin and insulin were dispersed in physiologically acceptable porous powdery or crystalline polyvalent metal compounds as the carrier and the resulting compositions were administered orally as enteric coated preparations, those peptides were found to be extremely well absorbed. In fact, the bioavailability was equivalent to that obtained by conventional injections, and thus the preparations were found to be clinically useful. The present inventor completed the invention based on these findings.

Accordingly, the present invention has an object of providing an orally administrable composition that allows excellent absorption of physiologically active peptides, which are normally poorly absorbed via the oral route, with improved pharmaceutical stability of those peptides.

### Disclosure of the Invention

The present invention provides a pharmaceutical composition for oral administration prepared as an enteric coated preparation, wherein an effective dose of physiologically active peptide is dispersed in a physiologically acceptable porous powdery or crystalline polyvalent metal compound carrier having a mean particle diameter of 500 µm or less.

The physiologically acceptable porous powdery or crystalline polyvalent metal compound carrier having a mean particle diameter of 500 µm or less includes such particles as those having a mean diameter of 5,000 µm or less, e.g., 2,000 to 3,000 µm, as a result of regranulation together with disintegrator(s).

A preferable mode of the invention provides a pharmaceutical composition that allows physiologically active peptides such as peptide hormones, opioid peptides, physiologically active proteins and enzyme proteins, but particularly peptide hormones, to be well absorbed via the oral route.

Another preferable mode of the invention provides a pharmaceutical composition that employs a divalent or higher valency metal compound, e.g., aluminum, calcium, magnesium, silicon, iron, or zinc compound and the like as the carrier to disperse therein an effective dose of physiologically active peptide.

The pharmaceutical composition of the present invention allows physiologically active peptides, which are normally poorly absorbed after oral administration, to be well absorbed via the oral route. Although efforts had been made to develop orally administrable preparations of physiologically active peptides, clinically applicable preparations had not been successfully developed. However, the pharmaceutical composition according to the present invention solved this problem and hence the invention is expected to provide great benefits in medical practice.

### Best Mode for Carrying Out the Invention

Physiologically active peptides to be contained in the pharmaceutical compositions of the present invention include peptide hormones, opioid peptide, physiologically active proteins and enzyme proteins, among which peptide hormones are preferable.

Peptide hormones include calcitonin, insulin, thyrotropin-releasing hormone (TRH) (for example, Thyroliberin), luteinizing hormone-releasing hormone (LH-RH) (for example, buserelin), LH-RH antagonists, somatostatin, adrenocorticotropic hormone (ACTH), adrenocorticotropic hormone-releasing hormone (CRH) (for example, Corticoliberin), growth hormone-releasing hormone (GH-RH) (for example, Somatorelin), gonadotropin (gonadotropic hormone), gonadotropin-releasing hormone (GnRH) (for example, Gonadoliberin), parathyroid hormone (PTH), thyroid-stimulating hormone (TSH), growth hormone (for example, somatotropin), prolactin (mammotropic hormone), follicle-stimulating hormone (FSH), glucagon, vasopressin, somatostatin (somatotropin-inhibiting hormone), parathormone (parathyroid hormone), angiotensin, gastrin, secretin, melanocyte-stimulating hormone, oxytocin, protirelin, corticotropin, thyrotropin (thyroid-stimulating hormone), G-CSF, erythropoietin, and superoxide dismutase (SOD).

In addition, various types of endorphins, interferons, interleukins, urokinase, lysozymes, and vaccines are also included.

Physiologically active peptides are not limited to those mentioned above, and other peptides which can be combined with the specific carrier and administered orally as enteric coated preparations may also be used to make the compositions of the present invention.

Among the above-mentioned physiologically active peptides, peptide hormones are preferable, and especially calcitonin, insulin, parathyroid hormone (PTH), buserelin (luteinizing hormone-releasing hormone: LH-RH), somatotropin (growth hormone: GH), gonadotropin (gonadotropic hormone), gonadotropin-releasing hormone (GnRH) and the like are preferable.

Calcitonin includes salmon calcitonin, human calcitonin, salmon-human chimera calcitonin, swine calcitonin, fowl calcitonin, bovine calcitonin, eel calcitonin, and the like. These calcitonins occur naturally, are extractable, and are commercially available. Any of these calcitonins may be used for the present invention.

Parathyroid hormone (PTH) includes human PTH, bovine PTH, swine PTH and the like, and among these human PTH (1-34) is preferable. Gonadotropin-releasing hormone (GnRH) and its analogues, which enhance secretion of LH, include buserelin acetate. Among growth hormones (GH), human growth hormone (HGH) is preferable.

In the present invention, the carrier to be used to disperse physiologically active peptide therein is polyvalent metal compound.

The polyvalent metal compounds suitable as the carrier are divalent or higher valency metal compounds, and more specifically such compounds include aluminum compounds, calcium compounds, magnesium compounds, silicon compounds, iron compounds, zinc compounds and the like. Some of these polyvalent metal compounds are powdery or crystalline compounds which are pharmaceutically used as excipients, stabilizers, fillers, binders, dispersants, disintegrators, lubricants, adsorbents and the like. These compounds have not been known to be useful as carriers of enteric coated preparations for oral administration of physiologically active peptides.

However, investigations by the present inventor have newly shown that these polyvalent metal compounds are highly useful as a carrier for oral administration of physiologically active peptides, especially as enteric coated preparations, because these compounds protect the physiologically active peptides from degradation by gastrointestinal proteases and increases absorption of said peptides.

Aluminum compounds suitable as the carrier include dried aluminum hydroxide gel, aluminum chlorhydroxide, synthetic aluminum silicate, light aluminum oxide, colloidal hydrous aluminum silicate, aluminum magnesium hydroxide, aluminum hydroxide, aluminum hydroxide gel, aluminum sulfate, dihydroxy aluminum acetate, aluminum stearate, natural aluminum silicate, aluminum monostearate, aluminum potassium sulfate and the like.

Calcium compounds include apatite, hydroxyapatite, calcium carbonate, calcium disodium edetate, calcium chloride, calcium citrate, calcium glycerophosphate, calcium gluconate, calcium silicate, calcium oxide, calcium hydroxide, calcium stearate, calcium tertiary phosphate, calcium lactate, calcium pantothenate, calcium oleate, calcium palmitate, calcium D-pantothenate, calcium alginate, calcium phosphate anhydride, calcium hydrogen phosphate, calcium dihydrogen phosphate, calcium acetate, calcium saccharate, calcium sulfate, calcium monohydrogen phosphate, calcium p-aminosalicylate, bio-calcilutite compounds and the like.

Magnesium compounds include magnesium L-asparginate, magnesium chloride, magnesium gluconate, magnesium silicate aluminate, magnesium silicate, magnesium oxide, magnesium hydroxide, magnesium stearate, magnesium carbonate, magnesium metasilicate aluminate, magnesium sulfate, sodium magnesium silicate, synthetic sodium magnesium silicate and the like.

Silicon compounds include hydrous silicon dioxide, light silicic acid anhydride, synthetic hydrotalcite, diatomaceous earth, and silicon dioxide. Iron compounds include iron sulfate, and zinc compounds include zinc chloride, zinc stearate, zinc oxide, zinc sulfate and the like.

The carrier in the pharmaceutical composition of the present invention may be a single compound or a combination of two or more compounds.

Polyvalent metal compounds for use as the carrier should have a mean particle diameter of 500 µm or less, preferably 250 µm or less, and more preferably 0.1 to 100 µm, and should be porous powders or crystals. Fine particles which have a mean diameter of 5,000 µm or less, e.g., 2,000 to 3,000 µm as a result of regranulation with disintegrator(s) can also be used as the carrier if such particles are porous powders or crystals in structure.

Such disintegrators include starches such as corn starch, wheat starch, and potato starch, sodium carboxymethyl starch, modified starches such as primogel, carboxymethyl cellulose, calcium carboxymethyl cellulose, low-substituted hydroxypropyl cellulose and the like.

The particles granulated with such disintegrator(s) are readily disintegrated after oral administration and therefore the active ingredient, i.e., physiologically active peptide dispersed in the carrier as fine particles, becomes absorbable.

Among the above-mentioned polyvalent metal compounds, especially calcium compounds (hydroxyapatite, calcium carbonate, and calcium lactate), a magnesium compound (magnesium stearate) and an aluminum compound (aluminum hydroxide) have been found to provide favorable results.

When physiologically active peptide is dispersed in such porous powdery or crystalline compounds and enclosed in the pores, degradation by proteases is prevented, and thus the physiologically active peptide can be efficiently absorbed from the intestinal mucosa when administered as an oral preparation.

Among the polyvalent metal compounds as the specific carrier in the pharmaceutical composition of the present invention, hydroxyapatite (Ca₁₀(PO₄)₆(OH)₂), which is one of calcium compounds, is a major inorganic component of the bones and teeth of animals and has been used to coat the surface of artificial bone made of ceramic materials for medical purposes, but it has not been investigated as a carrier for oral preparations.

As a carrier similar to hydroxyapatite, bio-calcilutite compounds can also be used, which include calcium pyrophosphate crystals (Ca₂P₂O₇·2H₂O), calcium secondary phosphate crystals (CaHPO₄·2H₂O), octacalcium phosphate crystals (Ca₈H₂(PO₄)₆·5H₂O), tricalcium phosphate (Ca₃(PO₄)₂), calcium oxalate crystals (CaC₂O₄·H₂O) and the like. Hydroxyapatites employable as the carrier include synthetic hydroxyapatite produced by the dry synthesis or wet synthesis method and bio-hydroxyapatite produced from bones or teeth by removing organic substances.

Further investigations by the present inventor have shown that when non-steroidal anti-inflammatory/analgesic agents are incorporated into the orally administrable compositions containing physiologically active peptides dispersed in the above-mentioned polyvalent metal compound carrier, oral absorption of the physiologically active peptides, which usually show poor absorption orally, is further enhanced. That is, non-steroidal anti-inflammatory/analgesic agents have newly been found to enhance the oral absorption of physiologically active peptides.

Accordingly, the present invention also proposes the use of non-steroidal anti-inflammatory/analgesic agents as an oral absorption enhancer of physiologically active peptides.

Non-steroidal anti-inflammatory/analgesic agents as an absorption enhancer of physiologically active peptides include those which are widely known, e.g., indomethacin, diclofenac, sulindac, ibuprofen, ketoprofen, flurbiprofen, naproxen, fenoprofen, oxaprozin, loxoprofen, mefenamic acid, 4-biphenylyl acetic acid, piroxicam and the like. Their content may range from 5 to 20 weight %, preferably about 8 to 15 weight %, of the total weight of the composition.

Further investigations by the present inventor have shown that when gelatin was added to the physiologically active peptide composition (preparation) for oral administration set forth hereinabove, the stability of the preparation was surprisingly improved. For example, insulin is only available as injectable preparations which have to be dissolved immediately before use and to be stored in a cold place. However, it has been shown that the stability of the insulin-containing pharmaceutical composition for oral administration according to the present invention is markedly improved by addition of gelatin.

Accordingly, another aspect of the present invention relates to a pharmaceutical composition for oral administration containing physiologically active peptide dispersed in the carrier proposed by the present inventor, and further containing gelatin as a stabilizer, and especially proposes the use of gelatin as a stabilizer for oral preparations containing physiologically active peptides.

It has been found that the content of gelatin may range from 0.05 to 5 weight % of the total weight of the pharmaceutical composition, and favorable results have been obtained at a content of 0.1 to 2 weight %.

The effective dose of the physiologically active peptide to be contained in the pharmaceutical composition for oral administration according to the present invention depends on the individual active substance, the disease to be treated, the frequency of oral administration desired, the therapeutic efficacy required, etc., and can be determined by comparison with the bioavailability of known preparations containing the same peptide.

The effective content of physiologically active peptide in the composition for oral administration according to the present invention may be, for example, 0.005 to 30%, preferably 0.01 to 20%, and more preferably 0.1 to 5.0%, per 100% total weight of a powder preparation.

More specifically, it is preferable that the calcitonin content is 200 IU/30 mg as a composition, PTH (1-34) content is 60 µg/30 mg or 90 µg/40 mg as a composition, and gonadotropin-releasing hormone (GnRH) content is 50 µg/30 mg as a composition.

It has been shown that favorable oral absorption is obtained when the content of the polyvalent metal compound as the carrier, e.g., hydroxyapatite, calcium carbonate, calcium lactate, magnesium stearate, aluminum hydroxide and the like, is 70 to 99.995%, preferably 80 to 99.99%, and more preferably 95 to 99.9%, per 100% total weight of the composition.

The pharmaceutical composition for oral administration according to the present invention can be prepared by appropriately applying conventional pharmaceutical technology.

For example, when the carrier is a calcium compound such as hydroxyapatite, calcium carbonate or calcium lactate; a magnesium compound such as magnesium stearate; or an aluminum compound such as aluminum hydroxide, the pharmaceutical composition can be prepared by mixing the carrier and physiologically active peptide. This mixing may be done, for example, in a mortar by applying pressure or shear force.

It is desirable for the physiologically active peptide to be as fine a powder as possible, and the mean particle diameter should usually be 20 µm or less, preferably 10 µm or less.

Thus, the intended pharmaceutical composition for oral administration can be obtained. Since the active substance of the composition is physiologically active peptide that is absorbed in the intestines, the pharmaceutical composition is to be made into an enteric coated preparation in order to avoid the effect of proteases. The enteric coated preparation is preferably prepared by filling the peptide-containing composition in enteric coating capsules or by making the composition into tablets with enteric coating.

The composition of the present invention may also contain conventional excipients, stabilizers, fillers, binders, dispersants, disintegrators, lubricants, adsorbents and the like.

The specific effects offered by the pharmaceutical composition for oral administration according to the present invention are shown below by way of Test Examples.

### [Test Examples]

### Test Example 1

### Preparation of the composition of the present invention:

Buserelin, an LH-RH preparation, was selected as a physiologically active peptide, and a calcium compound, i.e., calcium carbonate, was selected as the carrier to prepare the enteric coated pharmaceutical composition for oral administration according to the present invention.

Both components were mixed to make a composition in which the buserelin content was 300 µg/30 mg, and the resultant mixture was filled in enteric coating capsules.

The particle diameter of calcium carbonate as the carrier was 40 to 45 µm.

### Absorption after oral administration:

A single oral dose of the above-mentioned composition was administered to three cynomolgus monkeys. Their blood samples were collected before dosing, and at 30, 45, 60, 120, and 150 minutes after dosing to determine the blood buserelin level by the direct method.

The results are shown in Table 1.

**Table 1**

| Blood buserelin level | | | |
|---|---|---|---|
| Time (min) | Blood buserelin level (pg/ml) | | |
| | Animal No. | | |
| | No. 1 | No. 2 | No. 3 |
| Before dosing | 30 or less | 30 or less | 30 or less |
| 30 | 30 or less | 30 or less | 30 or less |
| 45 | 30 or less | 30 or less | 30 or less |
| 60 | 30 or less | 30 or less | 30 or less |
| 120 | 66.0 | 107.0 | 53.0 |
| 150 | 81.0 | 193.0 | 70.0 |

The results given in the table clearly show that buserelin was well absorbed after oral administration without degradation by gastrointestinal proteases.

Accordingly, it has been demonstrated that the enteric coated composition using calcium carbonate as the specific carrier (vehicle) according to the present invention yields an absorption level of buserelin into the blood after oral administration which is equivalent to that generally obtained after injection. In particular, the composition allows a long-lasting absorption of physiologically active peptide, and therefore the clinical applicability would be wider as compared with injectable preparations.

### Test Example 2

### Preparation of the composition of the present invention:

Insulin was selected as a physiologically active peptide, and a calcium compound (calcium stearate) or a magnesium compound (magnesium stearate or magnesium oxide) was selected as the carrier to prepare the enteric coated composition for oral administration according to this invention.

Both components were mixed to make a composition in which the insulin content was 60 IU/150 mg, and the resultant mixture was filled in enteric coating capsules.

The particle diameter of the carrier was 40 to 45 µm.

### Absorption after oral administration:

A single oral dose of the above-mentioned composition was administered to two cynomolgus monkeys weighing 5 kg. Their blood samples were collected before dosing, and at 30, 60, 90, 120, 180, 240, 300, and 360 minutes after dozing to determine the blood insulin level.

The results are shown in Tables 2 to 4.

**Table 2**

| Blood insulin level | | | | |
|---|---|---|---|---|
| Time (min) | Blood insulin level (µg/ml) | | | |
| | Carrier: calcium stearate | | | |
| | Animal No. | | | |
| | No. 4 | No. 5 | Mean | SD |
| 0 | 0 | 9 | 9.0 | 0 |
| 30 | 10 | 43 | 26.5 | 23.3345 |
| 60 | 14 | 23 | 18.5 | 6.36400 |
| 90 | 11 | 15 | 13.0 | 2.82843 |
| 120 | 5 | 16 | 10.5 | 7.77818 |
| 180 | 3 | 11 | 7.0 | 5.65685 |
| 240 | 2 | 13 | 7.5 | 7.77817 |
| 300 | 4 | 15 | 9.5 | 7.77817 |
| 360 | 6 | 14 | 10.0 | 5.65685 |

**Table 3**

| Blood insulin level | | | | |
|---|---|---|---|---|
| Time (min) | Blood insulin level(µg/ml) | | | |
| | Carrier: magnesium stearate | | | |
| | Animal No. | | | |
| | No. 6 | No. 7 | Mean | SD |
| 0 | 21 | 19 | 20.0 | 1.41421 |
| 30 | 36 | 47 | 41.5 | 7.77817 |
| 60 | 33 | 114 | 73.5 | 57.2756 |
| 90 | 24 | 58 | 41.0 | 24.0416 |
| 120 | 43 | 78 | 60.5 | 24.7487 |
| 180 | 23 | 68 | 45.5 | 31.8198 |
| 240 | 37 | 33 | 35.0 | 2.82842 |
| 300 | 28 | 22 | 25.0 | 4.24264 |
| 360 | 23 | 30 | 26.5 | 4.94974 |

**Table 4**

| Blood insulin level | | | | |
|---|---|---|---|---|
| Time (min) | Blood insulin level(µg/ml) | | | |
| | Carrier: magnesium oxide | | | |
| | Animal No. | | | |
| | No. 8 | No. 9 | Mean | SD |
| 0 | 11 | 11 | 11.0 | 0 |
| 30 | 16 | 39 | 27.5 | 16.2634 |
| 60 | 6 | 60 | 33.0 | 38.1837 |
| 90 | 14 | 33 | 23.5 | 13.4350 |
| 120 | 5 | 25 | 15.0 | 14.1421 |
| 180 | 3 | 20 | 11.5 | 12.0208 |
| 240 | 3 | 19 | 11.0 | 11.3137 |
| 300 | 5 | 10 | 7.5 | 3.53553 |
| 360 | 3 | 12 | 7.5 | 6.36396 |

The results given in the tables clearly show that insulin was well absorbed after oral administration without degradation by gastrointestinal proteases. Since insulin has conventionally been only available as injectable preparations, the effect of the present invention should be considerable.

### Test Example 3

### Preparation of the composition of the present invention:

PTH (1-34) was selected as a physiologically active peptide, and a calcium compound (calcium carbonate or AW ceramic which is an apatite compound) or a magnesium compound (magnesium oxide) was selected as the carrier to prepare the enteric coated pharmaceutical composition for oral administration according to the present invention.

Both components were mixed to make a composition in which the PTH (1-34) content was 600 µg/200 mg, and the resultant mixture was filled in enteric coating capsules.

The particle diameter of calcium carbonate, AW ceramics, and magnesium oxide used as the carrier was 40 to 45 µm.

### Absorption after oral administration:

A single oral dose of the above-mentioned composition was administered to two cynomolgus monkeys weighing 5 kg. Their blood samples were collected before dosing, and at 30, 60, 90, 120, 180, 240, 300, and 360 minutes after dosing to determine the blood PTH (1-34) level by using ELISA with a 1-34PTH2 antibody.

The results are shown in Tables 5 to 7.

**Table 5**

| Blood PTH (1-34) level | | | | |
|---|---|---|---|---|
| Time (min) | Blood PTH (1-34) level(pg/ml) | | | |
| | Carrier: calcium carbonate | | | |
| | Animal No. | | | |
| | No. 10 | No.11 | Mean | SD |
| 0 | 36 | 15 | 25.5 | 14.8492 |
| 30 | 155 | 10 | 82.5 | 102.530 |
| 60 | 70 | 14 | 42.0 | 39.5979 |
| 90 | 47 | 10 | 28.5 | 26.1629 |
| 120 | 34 | 9 | 21.5 | 17.6776 |
| 180 | 36 | 11 | 23.5 | 17.6776 |
| 240 | 32 | 16 | 24.0 | 11.3137 |
| 300 | 44 | 26 | 35.0 | 12.7279 |
| 360 | 28 | 25 | 26.5 | 2.12132 |

**Table 6**

| Blood PTH (1-34) level | | | | |
|---|---|---|---|---|
| Time (min) | Blood PTH (1-34) level(pg/ml) | | | |
| | Carrier: AW ceramic | | | |
| | Animal No. | | | |
| | No. 12 | No.13 | Mean | SD |
| 0 | 27 | 21 | 24.0 | 4.24264 |
| 30 | 16 | 17 | 16.5 | 0.70710 |
| 60 | 17 | 14 | 15.5 | 2.12132 |
| 90 | 21 | 17 | 19.0 | 2.82842 |
| 120 | 27 | 13 | 20.0 | 9.89949 |
| 180 | 28 | 20 | 24.0 | 5.65685 |
| 240 | 24 | 24 | 24.0 | 0 |
| 300 | 25 | 31 | 28.0 | 4.24264 |
| 360 | 30 | 30 | 30.0 | 0 |

**Table 7**

| Blood PTH (1-34) level | | | | |
|---|---|---|---|---|
| Time (min) | Blood PTH (1-34) level(pg/ml) | | | |
| | Carrier: magnesium oxide | | | |
| | Animal No. | | | |
| | No. 14 | No. 15 | Mean | SD |
| 0 | 16 | 22 | 19.0 | 4.24264 |
| 30 | 82 | 15 | 48.5 | 47.3761 |
| 60 | 810 | 20 | 415 | 558.614 |
| 90 | 118 | 13 | 65.5 | 74.2462 |
| 120 | 48 | 10 | 29.0 | 26.8700 |
| 180 | 29 | 13 | 21.0 | 11.3137 |
| 240 | 22 | 11 | 16.5 | 7.77817 |
| 300 | 33 | 14 | 23.5 | 13.4350 |
| 360 | 35 | 12 | 23.5 | 16.2634 |

The results given in the tables clearly show that PTH (1-34) was well absorbed after oral administration.

### Example 4

### Preparation of a pharmaceutical using this invention:

Insulin was selected as a physiologically active peptide, a magnesium compound (magnesium oxide) was selected as the carrier, and a non-steroidal anti-inflammatory agent (piroxicam) was used as an absorption enhancer to prepare an enteric coated pharmaceutical composition for oral administration according to the present invention.

The peptide and the carrier were mixed to make a composition in which the insulin content was 60 IU/150 mg, and to this composition was added 10 mg of piroxicam. Then, the resultant mixture was filled in enteric coating capsules.

The particle diameter of the carrier was 40 to 45 µm.

### Absorption after oral administration:

A single oral dose of the above-mentioned composition was administered to two cynomolgus monkeys weighing 5 kg. Their blood samples were collected before dosing, and at 30, 60, 90, 120, 180, 240, 300, and 360 minutes after dosing to determine the blood concentration of insulin.

The results are shown in Table 8.

**Table 8**

| Blood insulin level | | | | |
|---|---|---|---|---|
| Time (min) | Blood insulin level(µg/ml) | | | |
| | Carrier: magnesium oxide/piroxicam | | | |
| | Animal No. | | | |
| | No. 16 | No. 17 | Mean | SD |
| 0 | 31 | 17 | 24.0 | 9.89949 |
| 30 | 71 | 19 | 45.0 | 36.7695 |
| 60 | 32 | 26 | 29.0 | 4.24264 |
| 90 | 28 | 16 | 22.0 | 8.48528 |
| 120 | 30 | 15 | 22.5 | 10.6066 |
| 180 | 39 | 21 | 30.0 | 12.7279 |
| 240 | 21 | 13 | 17.0 | 5.65685 |
| 300 | 21 | 22 | 21.5 | 0.70710 |
| 360 | 29 | 9 | 19.0 | 14.1421 |

The results given in the table clearly show that insulin was well absorbed without degradation by gastrointestinal proteases after oral administration, and that addition of piroxicam enhanced its absorption.

### Test Example 5

### Preparation of the composition of the present invention:

PTH (1-34) was selected as a physiologically active peptide, a magnesium compound (magnesium oxide) was selected as the carrier, and a non-steroidal anti-inflammatory agent (diclofenac) was used as an absorption enhancer to prepare enteric coated pharmaceutical composition for oral administration according to the present invention.

The peptide and the carrier were mixed to make a composition in which the PTH (1-34) content was 600 µg/200 mg, and to this composition was added 10 mg of diclofenac. Then, the resultant mixture was filled in enteric coating capsules.

The particle diameter of magnesium oxide used as the carrier was 40 to 45 µm.

### Absorption after oral administration:

A single oral dose of the above-mentioned composition was administered to two cynomogus monkeys weighing 5 kg. Their blood samples were collected before dosing, and at 30, 60, 90, 120, 180, 240, 300, and 360 minutes after dosing to determine the blood PTH (1-34) level by using ELISA with a 1-34PTH2 antibody.

The results are shown in Table 9.

**Table 9**

| Blood PTH (1-34) level | | | | |
|---|---|---|---|---|
| Time (min) | Blood PTH (1-34) level (pg/ml) | | | |
| | Carrier: magnesium oxide/diclofenac | | | |
| | Animal No. | | | |
| | No.18 | No. 19 | Mean | SD |
| 0 | 53 | 25 | 39.0 | 19.7989 |
| 30 | 17 | 16 | 16.0 | 0.70710 |
| 60 | 17 | 16 | 16.5 | 0.70710 |
| 90 | 14 | 19 | 16.5 | 3.53553 |
| 120 | 15 | 19 | 17.0 | 2.82842 |
| 180 | 14 | 16 | 15.0 | 1.41421 |
| 240 | 21 | 17 | 19.0 | 2.82842 |
| 300 | 16 | 13 | 14.5 | 2.12132 |
| 360 | 16 | 21 | 18.5 | 3.53553 |

The results given in the table clearly show that PTH (1-34) was well absorbed without degradation by gastrointestinal proteases after oral administration, and that addition of diclofenac enhanced its absorption.

### Example 6: Stability test

### Preparation of the composition of the present invention:

Insulin was selected as a physiologically active peptide, and a calcium compound, i.e., calcium carbonate, was selected as the carrier and gelatin was added as a stabilizer to prepare the enteric coated pharmaceutical composition for oral administration according to the present invention.

Both components were mixed to make compositions in which the insulin content was 24 IU/40 mg, and gelatin was added at 0.3% or 1.0% of the weight of the composition.

### Stability test:

The compositions prepared as above were stored in a constant-temperature chamber at 40°C, and the residual percentage of insulin was determined after 10 days and 1 month.

The tests were performed on 3 samples at each concentration of gelatin, and the mean residual percentage of insulin was determined, considering the initial insulin content to be 100%.

The results are shown in Table 10.

**Table 10**

| Results of the stability test (Residual percentage of insulin) | | |
|---|---|---|
| Carrier/amount, storage conditions | after 10 days | after 1 month |
| Calcium carbonate/0.3% gelatin, 40°C | 100.44% | 95.13% |
| Calcium carbonate/1.0% gelatin, 40°C | 97.42% | 93.72% |

The results in the table clearly show that addition of gelatin maintained the stability of insulin.

The stability is considered very high as compared with that of conventional bulk insulin powder, which shows a residual percentage of as low as about 10% after 1-month storage under severe conditions at 40°C.

The following are formulation examples of the composition according to the present invention, which, however, are non-limiting.

### Powder composition 1: calcitonin composition

A calcitonin composition was prepared from the following components:

| | |
|---|---|
| Calcitonin | 6540 IU |
| Calcium carbonate | the rest part |
| | 1,000 mg |

A fine powder comprising the above-mentioned components was filled in enteric coating capsules for oral administration.

### Powder composition 2: PTH (1-34) composition

A PTH (1-34) composition was prepared from the following components:

| | |
|---|---|
| PTH (1-34) | 1,962 µg |
| Aluminum hydroxide | the rest part |
| | 1,000 mg |

A fine powder comprising the above-mentioned components was filled in enteric coating capsules for oral administration.

### Powder composition 3: buserelin composition

A buserelin composition was prepared from the following components:

| | |
|---|---|
| Buserelin | 10,000 µg |
| Calcium carbonate (or aluminum hydroxide) | the rest part |
| | 1,000 mg |

A fine powder comprising the above-mentioned components was filled in enteric coating capsules for oral administration.

### Industrial Applicability

As mentioned above, the present invention makes it feasible to enhance the absorption of physiologically active peptides, which are normally poorly absorbed after oral administration, by using physiologically acceptable porous powdery or crystalline polyvalent metal compounds as the carrier for drug delivery.

Especially, the compositions of the present invention wherein physiologically active peptides, including calcitonin, insulin, buserelin, glucagon, somatotropin and the like are dispersed in polyvalent metal compounds such as calcium carbonate, calcium lactate, magnesium stearate and aluminum hydroxide as the specific carrier, can be administered via the oral route as enteric coated preparations, thereby enhancing absorption of such peptides so that effective clinical therapy becomes practicable.

The specific carrier used in the present invention can also enhance absorption of not only physiologically active peptides but also other active substances which are normally poorly absorbed via the oral route. Such substances include vitamins such as vitamin B₁₂, anti-allergic agents, immunosuppressants, parenteral antibiotics/antimicrobials, antitumor agents, drugs for treating osteoporosis, and the like. Thus, the present invention can provide considerable benefits for medical care.

## Claims

1. A pharmaceutical composition for oral administration prepared as an enteric coated preparation, wherein an effective dose of physiologically active peptide is dispersed in a physiologically acceptable porous powdery or crystalline polyvalent metal compound carrier with a mean particle diameter of 500 µm or less.

2. A pharmaceutical composition for oral administration according to claim 1, in which the physiologically acceptable porous powdery or crystalline polyvalent metal compound carrier with a mean particle diameter of 500 µm or less is granulated together with disintegrator(s) to make fine particles with a mean diameter of 5,000 µm or less.

3. A pharmaceutical composition for oral administration according to claim 1, in which the physiologically active peptide is selected from the group consisting of peptide hormone, opioid hormone, physiologically active protein and enzyme protein.

4. A pharmaceutical composition for oral administration according to claim 1, in which the physiologically active peptide is selected from the group consisting of calcitonin, insulin, opioid peptide, thyrotropin-releasing hormone (TRH), luteinizing hormone-releasing hormone (LH-RH), LH-RH antagonist, somatostatin (growth hormone-inhibiting hormone), adrenocorticotropic hormone (ACTH), adrenocorticotropic hormone-releasing hormone (CRH), growth hormone-releasing hormone (GH-RH), gonadotropin (gonadotropic hormone), gonadotropin-releasing hormone (GnRH), parathyroid hormone (PTH), growth hormone (GH), somatotropin, prolactin (mammotropic hormone), follicle-stimulating hormone (FSH), glucagon, and vasopressin.

5. A pharmaceutical composition for oral administration according to claim 1, in which the polyvalent metal compound carrier is divalent or higher valency metal compound selected from the group consisting of aluminum compound, calcium compound, magnesium compound, silicon compound, iron compound, and zinc compound.

6. A pharmaceutical composition for oral administration according to claim 1, in which the polyvalent metal compound carrier is aluminum compound selected from the group consisting of dried aluminum hydroxide gel, aluminum chlorhydroxide, synthetic aluminum silicate, light aluminum oxide, colloidal hydrous aluminum silicate, aluminum magnesium hydroxide, aluminum hydroxide, aluminum hydroxide gel, aluminum sulfate, dihydroxy aluminum acetate, aluminum stearate, natural aluminum silicate, aluminum monostearate, and aluminum potassium sulfate.

7. A pharmaceutical composition for oral administration according to claim 1, in which the polyvalent metal compound carrier is calcium compound selected from the group consisting of apatite, hydroxyapatite, calcium carbonate, calcium disodium edetate, calcium chloride, calcium citrate, calcium glycerophosphate, calcium gluconate, calcium silicate, calcium oxide, calcium hydroxide, calcium stearate, calcium tertiary phosphate, calcium lactate, calcium pantothenate, calcium oleate, calcium palmitate, calcium D-pantothenate, calcium alginate, calcium phosphate anhydride, calcium hydrogen phosphate, calcium dihydrogen phosphate, calcium acetate, calcium saccharate, calcium sulfate, calcium monohydrogen phosphate, calcium p-aminosalicylate, and bio-calcilutite compound.

8. A pharmaceutical composition for oral administration according to claim 1, in which the polyvalent metal compound carrier is magnesium compound selected from the group consisting of magnesium L-asparginate, magnesium chloride, magnesium gluconate, magnesium silicate aluminate, magnesium silicate, magnesium oxide, magnesium hydroxide, magnesium stearate, magnesium carbonate, magnesium metasilicate aluminate, magnesium sulfate, sodium magnesium silicate, and synthetic sodium magnesium silicate.

9. A pharmaceutical composition for oral administration according to claim 1, in which the polyvalent metal compound carrier is silicone compound selected from the group consisting of hydrous silicon dioxide, light silicic acid anhydride, synthetic hydrotalcite, diatomaceous earth, and silicon dioxide.

10. A pharmaceutical composition for oral administration as in any one of claims 1 to 9, which further contains a non-steroidal anti-inflammatory/analgesic agent as an absorption enhancer.

11. Use of a non-steroidal anti-inflammatory/analgesic agent as an absorption enhancer in the pharmaceutical composition for oral administration as in any one of claims 1 to 9.

12. A pharmaceutical composition for oral administration as in any one of claims 1 to 9, which further contains gelatin as a stabilizer.

13. Use of gelatin as a stabilizer in the pharmaceutical composition for oral administration as in any one of claims 1 to 9.
